# EUROPÄISCHE PATENTSCHRIFT

(11) **EP 2 575 664 B1**
(45) Veröffentlichungstag und Bekanntmachung des Hinweises auf die Patenterteilung: **13.02.2019**
(21) Anmeldenummer: 11725668.5
(22) Anmeldetag: 06.06.2011
(51) Int. Cl.: G02B 21/00, G02B 25/02, A61B 90/20, A61B 17/24

(54) **MEDIZINISCHE VERGRÖßERUNGSVORRICHTUNG**
MEDICAL MAGNIFICATION DEVICE
DISPOSITIF D'AGRANDISSEMENT MÉDICAL

(30) Priorität: 07.06.2010 DE 202010007662 U
(43) Veröffentlichungstag der Anmeldung: 10.04.2013
(73) Patentinhaber: Jurkat, Klaus-Peter, 13591 Berlin (DE)
(72) Erfinder: Jurkat, Klaus-Peter, 13591 Berlin (DE)
(74) Vertreter: Sperling, Thomas
(86) Internationale Anmeldenummer: PCT/EP2011/059280
(87) Internationale Veröffentlichungsnummer: WO 2011/154350

(56) Entgegenhaltungen:
- WO-A1-2005/124421
- DE-A1- 10 357 496
- US-A- 6 086 228
- US-B1- 6 508 759

## Beschreibung

Die Erfindung betrifft eine Vorrichtung zur visuellen Vergrößerung von Objekten zur Verwendung in der Medizin, insbesondere der Zahnmedizin und Hals-Nasen-Ohren-Medizin, zur Diagnostik kleinster Details in kleinen und besonders tiefen Kavitäten.

Auf dem Anwendungsgebiet der Erfindung werden beispielsweise Lupenbrillen eingesetzt, die jedoch nur eine geringe Vergrößerung ermöglichen. Im Stand der Technik sind weiterhin Operationsmikroskope im medizinischen Bereich, insbesondere der Chirurgie und Mikrochirurgie, als Mikroskope mit etwa 6facher bis 40facher Vergrößerung bekannt. Die Operationsmikroskope weisen gegenüber einer Lupenbrille eine stärkere Vergrößerung auf, erfordern jedoch eine besonders ruhige Positionierung und meist eine sehr unbequeme und aufwendige Lagerung des Patienten.
Das Operationsmikroskop wird auch als technisches Hilfsmittel bei zahnmedizinischen Behandlungen eingesetzt, insbesondere bei Eingriffen, bei denen winzige Stellen im Zahn behandelt werden. Es bietet dabei den großen Vorteil der Sichtbarmachung von mit bloßem Auge unsichtbaren Strukturen.
Das bedeutendste Einsatzgebiet des Operationsmikroskops innerhalb der Zahnmedizin ist die Behandlung des Zahninneren mit Zahnmark und Zahnwurzel, auch als Wurzelbehandlung bekannt. Außerdem werden Operationsmikroskope für die Erkennung von Karies eingesetzt.
Für eine Inspektion eines Wurzelkanals mit einem Durchmesser von etwa 1 mm und einer Tiefe von etwa 12,5 mm ist die optische Auflösung bei Betrachtung mit bloßem Auge unzureichend. Durch die Verwendung herkömmlicher Lupenbrillen mit 2facher bis 4facher Vergrößerung wird die optische Auflösung bereits verbessert. Um gleichzeitig die Ausleuchtung zu optimieren, ist zusätzlich eine Stirnlampe notwendig. Mittels der Kombination einer Lupenbrille und einer Stirnlampe lässt sich der Wurzelkanal bis in eine Tiefe von etwa 5 mm ausreichend beurteilen. Für größere Wurzelkanaltiefen sind Operationsmikroskope mit nahezu paralleler Lichtführung notwendig.
Das Operationsmikroskop ist dabei oberhalb des Behandlungsstuhls mittels Schwenkarmen gehaltert und erfüllt verschiedene Funktionen. Die im Stand der Technik bekannten Operationsmikroskope sind binokular und demzufolge mit jeweils einem Vergrößerungsaufbau für jedes Auge ausgebildet. Herkömmliche Operationsmikroskope sind stufenlos in allen Dimensionen allerdings nur beschränkt kippbar, um sehr enge, verwinkelte Kanäle und Hohlräume, wie gekrümmte Wurzelkanäle, im Zahninneren einsehbar zu machen. Die optische Vergrößerung ist dabei meist variabel einstellbar, damit einerseits Feinheiten von Strukturen erkennbar sind, aber andererseits die Übersicht über das gesamte Operationsgebiet gewährleistet ist.

Aus der DE 296 04 848 U1 geht ein binokulares Operationsmikroskop für Eingriffe im Dentalbereich, speziell für Eingriffe an Zähnen und am Kiefer, hervor. Das Operationsmikroskop ist nach unten gerichtet angeordnet, sodass der Arzt durch die Okulare und eine Frontlinse in den Kieferbereich schauen kann. Die Beleuchtung des zu operierenden Objektes erfolgt durch die Frontlinse hindurch, wobei das Licht durch einen Lichtleiter geführt oder von einer Lichtquelle innerhalb des Mikroskopes ausgesendet wird.

Die binokulare Ausbildung erfordert eine vergleichsweise stabile Aufhängung des Mikroskops, die herkömmlich durch eine schwingungsgedämpfte, wackelfreie Deckenmontage realisiert wird. Die Objektivrichtung verläuft dabei vertikal von oben nach unten und kann nur unter einem großen konstruktiven Aufwand in seitliche Richtungen begrenzt geschwenkt werden. Der behandelnde Arzt hat zudem eine sehr genaue Position zu den beiden Okularen einzunehmen und konstant zu halten. Schon leichte Bewegungen und Drehungen des Kopfes führen zu Doppelbildern und anderen Beeinträchtigungen. Die Ausleuchtung des zu operierenden Bereiches ist durch den Arbeitsabstand und den Abstand von Lichtquelle zu Objektiv aus konstruktiven Gründen limitiert. Zudem ist in sehr schmalen und engen Kanälen ein stereoskopisches Sehen aus geometrischen Gründen nicht möglich. Die Einstellung der Arbeitsentfernung ist meist durch ein nicht variables Objektiv auf etwa 250 mm vorgegeben und muss durch aufwendige mechanische Konstruktionen eingestellt werden. Zudem erfordern die Operationen mit einem Operationsmikroskop eine optimale und präzise Lagerung des Patienten.
Alternativ dazu ist aus der DE 102 03 215 B4 ein Operationsmikroskop mit einem Objektiv mit Lichtquelle zur Objektbeleuchtung, einem Okular und einer Kamera bekannt, wobei das Okular ein zu untersuchendes Objekt zur visuellen Beobachtung wiedergibt und die Kamera das Objekt durch das Objektiv hindurch aufnimmt. Das Okular ist dabei zusammen mit einer Wiedergabevorrichtung gegenüber dem Objekt und der Kamera in der Bewegung entkoppelt ausgebildet. Das Okular ist dafür an einem in Bezug auf das Objektiv verstellbaren Okularstativ befestigt, das wiederum an einem das Objektiv und die Kamera tragenden Mikroskopstativ gehaltert ist. Das Objektiv und die Okularlage sind dadurch entkoppelt voneinander ausgebildet.
Auch in der DE 20 2006 020 039 U1 wird ein stereoskopisches Beobachtungsgerät mit einer Beleuchtungseinrichtung und variabler Blende zur Abbildung eines Objektes und/oder eines von einem Objekt erzeugten Zwischenbildes offenbart, wobei die variable Blende für einen Strahlengang der Beleuchtungseinrichtung vorgesehen ist. Die variable Blende, in der eine Lichtquelle integriert ist, ist zur Erzeugung einer bestimmten Beleuchtungsgeometrie bereichsweise ansteuerbar und/oder in ihrer Lage veränderbar.

US 6508759 beschreibt eine medizinische Vergrößerungsvorrichtung mit einer optischen Einheit mit einer optischen Achse, eine Beleuchtungseinrichtung und Befestigungselemente, wobei die optische Einheit am proximalen Ende ein stereoskopisches Okular aufweist, die Beleuchtungseinrichtung hohlzylinderförmig ausgebildet, am distalen Ende der optischen Einheit mit der optischen Einheit verbunden und koaxial zur optischen Achse angeordnet ist und ein konzentrisches Beleuchtungsmuster erzeugt. Die Befestigungselemente umfassen mit der optischen Einheit verbindbare Mittel, um die optische Einheit richtungsunabhängig zu verstellen.

Aufgabe der vorliegenden Erfindung ist es, eine Vorrichtung zur visuellen Vergrößerung von Objekten zur Verwendung in der Medizin, insbesondere der Zahnmedizin und der Hals-Nasen-Ohren-Medizin, zur Diagnostik kleinster Details in kleinen und besonders tiefen Kavitäten zur Verfügung zu stellen. Die Vorrichtung soll dabei derart ausgebildet sein, dass bei der Bedienung der Vorrichtung während einer Operation eine optimale Beleuchtung des zu untersuchenden Objektes gewährleistet ist. Besonders bei tiefen und engen Strukturen soll eine gute Ausleuchtung des zu operierenden Bereiches bei möglichst geringer Blendung und möglichst wenig Schatten erreicht werden. Die Vorrichtung soll sich im Bereich der Zahnmedizin, insbesondere für die Inspektion von Wurzelkanälen und Zahnzwischenräumen, eignen. Die Bedienung der Vorrichtung sollte einfach und ergonomisch sein. Weiterhin ist es ein Ziel, die Vorrichtung aus einer geringen Anzahl konstruktiv einfacher Komponenten und damit kostengünstig sowie mit geringem Aufwand herstellbar auszubilden.
Die Aufgabe wird erfindungsgemäß durch eine medizinische Vergrößerungsvorrichtung nach Anspruch 1 gelöst. Die Vorrichtung weist eine optische Einheit mit einer optischen Achse, eine Beleuchtungseinrichtung und Befestigungselemente auf, wobei die optische Einheit monokular ausgebildet ist und am proximalen Ende ein Okular aufweist. Unter dem proximalen Ende der optischen Einheit ist das dem Betrachter zugewandte Ende zu verstehen. Der Betrachter schaut folglich am proximalen Ende in die optische Einheit hinein.
Am distalen Ende, das heißt dem Betrachter abgewandten Ende, der optischen Einheit ist in Richtung der optischen Achse die hohlzylindrisch ausgebildete Beleuchtungseinrichtung vorgesehen, welche den Bereich in Richtung der optischen Achse ausleuchtet. Nach der Konzeption der Erfindung ist die Beleuchtungseinrichtung dabei koaxial zur optischen Achse angeordnet und fest mit der optischen Einheit verbunden. Die Beleuchtungseinrichtung erzeugt ein zur optischen Achse der optischen Einheit konzentrisches Beleuchtungsmuster, welches die vorteilhafte Ausleuchtung der zu untersuchenden Objekte gewährleistet. Die feste Verbindung zwischen der Beleuchtungseinrichtung und der optischen Einheit gewährleistet die Einstellung der Vorrichtung, insbesondere der optischen Einheit, ohne dass es einer zusätzlichen Einstellung der Beleuchtungseinrichtung bedarf. Die optische Einheit und die Beleuchtungseinrichtung werden vorteilhaft innerhalb eines Vorgangs eingestellt.
Die Befestigungselemente der erfindungsgemäßen Vergrößerungsvorrichtung weisen zudem mit der optischen Einheit verbindbar ausgebildete Mittel auf, welche eine richtungsunabhängige Verstellbarkeit der optischen Einheit in Bezug auf den Patienten ermöglichen. Unter der richtungsunabhängigen Verstellbarkeit ist dabei die unbeschränkte Bewegung der optischen Einheit in allen Richtungen zu verstehen. Damit ist die optische Einheit lageunabhängig, unbegrenzt in jeder beliebigen Position fixierbar.

Nach einer bevorzugten Ausgestaltung der Erfindung weist die Beleuchtungseinrichtung ringförmig um die optische Achse angeordnete Leuchtkörper in Form von LED auf. Die LED sind dabei gleichmäßig am Umfang der hohlzylindrisch ausgebildeten Beleuchtungseinrichtung und damit in ihrer Gesamtheit koaxial um die optische Achse verteilt. Unter einer koaxialen, gleichmäßig am Umfang verteilten Anordnung der LED ist eine Anordnung der LED im gleichen Abstand von der optischen Achse und demzufolge von der Mittelachse der Beleuchtungseinrichtung zu verstehen. Infolge der koaxialen Ausrichtung der Beleuchtungseinrichtung in Bezug auf die optische Einheit liegen die optische Achse und die Mittelachse der Beleuchtungseinrichtung übereinander. Die optische Achse der optischen Einheit und die Mittelachse der Beleuchtungseinrichtung sind identisch.
Die Beleuchtungseinrichtung ist vorteilhaft mit vier LED ausgebildet.

Die Vergrößerungsvorrichtung weist nach einer vorteilhaften Ausgestaltung der Erfindung elektrisch angetriebene Stellmotoren auf, welche mit einer Stromquelle elektrisch verbunden sind. Die Stellmotoren sind zum einen zur Fokussierung beziehungsweise zum Antrieb einer Fokussiereinrichtung der optischen Einheit und zum anderen zur Ausrichtung der optischen Einheit in Bezug auf die Position zum Patienten und/oder zur behandelnden Person mithilfe der Befestigungselemente, das heißt zur Lageänderung der optischen Einheit, vorgesehen.
Die optische Einheit, insbesondere die Beleuchtungseinrichtung sowie die Stellmotoren der Fokussiereinrichtung, und die Stellmotoren der Befestigungselemente sind bevorzugt mit einem elektrischen Energiespeicher, beispielsweise in Form einer Batterie, als Stromquelle verbunden. Die Beleuchtungseinrichtung und die Stellmotoren können als Verbraucher jedoch alternativ auch in Verbindung mit dem Stromnetz betreibbar ausgebildet sein. Alternativ zur Ausbildung mit elektrischen Stellmotoren sind die optische Einheit, insbesondere die Fokussiereinrichtung, sowie die Befestigungselemente auch manuell bedienbar, sodass sich die Stromversorgung der erfindungsgemäßen Vergrößerungsvorrichtung lediglich auf die Beleuchtungseinrichtung beschränkt.

Nach einer bevorzugten Weiterbildung der Erfindung ist die optische Einheit am proximalen Ende mit einer Bilderfassungsvorrichtung erweiterbar oder mit dem Okular austauschbar ausgebildet. Dabei ist die Bilderfassungsvorrichtung als Okular, Monitor und/oder Kamera zum Anzeigen sowie zur Aufnahme von Bildern in Form von Fotografien oder Filmen ausgebildet.

Die Fokussiereinrichtung der optischen Einheit ist bevorzugt für eine Fokussierung im Bereich zwischen 20 cm und 40 cm einstellbar ausgebildet. Die Fokussierung wird dabei direkt an der optischen Einheit durchgeführt.
Des Weiteren weist die optische Einheit für den Nahbereich vorteilhaft eine bis zu 12fache Vergrößerung auf, wobei das Optimum im Bereich der 8fachen bis 12fachen Vergrößerung liegt. Der Wert der Vergrößerung ist zudem zum einen durch die Variation des Abstandes zwischen der Vergrößerungsvorrichtung und dem zu untersuchenden Objekt und zum anderen mithilfe einer optischen Zoomeinrichtung einstellbar.

Nach einer weiteren Ausgestaltung der Erfindung umfassen die Mittel der Befestigungselemente zur Befestigung der optischen Einheit Halterungen, Gelenke und Feststelleinrichtungen. Die Befestigungselemente sind dabei bevorzugt aus Federarmhalterungen mit Scharniergelenken sowie mit mindestens einem Kugelgelenk ausgebildet, sodass die optische Einheit in der Objektivausrichtung unbegrenzt einstellbar ist.
Die Befestigungselemente sind während des Betriebes beziehungsweise der Behandlung oder Operation mit der optischen Einheit verbunden. Die optische Einheit mit der daran angeordneten Beleuchtungseinrichtung ist mittels der Befestigungselemente vorteilhaft am Behandlungsstuhl montierbar und schwenkbar gehaltert. Bei Nichtbenutzung ist die Vergrößerungsvorrichtung einfach demontierbar beziehungsweise aus dem Operationsbereich herausschwenkbar. Die Befestigungselemente sind sowohl vom Behandlungsstuhl als auch von der optischen Einheit zu lösen und in wenigen Einzelkomponenten verstaubar.
Alternativ ist die optische Einheit mit der Beleuchtungseinrichtung mittels der Befestigungselemente vorteilhaft auch an einer herkömmlichen Operationsleuchte montierbar beziehungsweise innerhalb der Operationsleuchte integrierbar ausgebildet. Je nach Anforderung des medizinischen Personals an die Operationsbereichsausleuchtung ist die erfindungsgemäße optische Einheit auch anstelle der Operationsleuchte betreibbar. Die optische Einheit wird dann die Operationsleuchte ersetzen.

Die erfindungsgemäße Lösung weist somit diverse Vorteile im Hinblick auf die Untersuchung von engen und tiefen Kavitäten, beispielsweise Zahnkavitäten, auf, die bisher lediglich mit aufwendig konstruierten und komplex bedienbaren sowie kostenintensiven Stereomikroskopen möglich sind. Die vorliegende Vorrichtung zur visuellen Vergrößerung von Objekten, insbesondere im zahnmedizinischen Bereich, gewährleistet die Untersuchung von Karies in Zahnzwischenräumen sowie Unregelmäßigkeiten in Wurzelkanälen von Zähnen, beispielsweise im Kanalverlauf, der Krümmung, versteckten Abzweigungen, Zementresten oder Ähnlichem bei optimaler Beleuchtung des zu untersuchenden Objektes und einfacher sowie ergonomischer Bedienung. Mit der erfindungsgemäßen, aus einer geringen Anzahl und konstruktiv einfachen Komponenten und damit geringem konstruktiven Aufwand herstellbaren Vorrichtung lässt sich die Behandlungsqualität steigern und/oder die Behandlungszeit verkürzen. Die Vorrichtung ist damit sowohl bei der Anschaffung als auch in der Unterhaltung sowie im Betrieb sehr effektiv und kostengünstig.

Weitere Einzelheiten, Merkmale und Vorteile der Erfindung ergeben sich aus der nachfolgenden Beschreibung von Ausführungsbeispielen mit Bezugnahme auf die zugehörigen Zeichnungen. Es zeigen:
- Fig. 1:: monokulare Vorrichtung zur visuellen Vergrößerung von Objekten in Seitenansicht,
- Fig. 2:: optische Einheit und Beleuchtungseinrichtung mit optischem Durchlass,
- Fig. 3:: Zahn mit angedeuteter äußerer Zahnkontur und Wurzelkanal in Seitenansicht und
- Fig. 4:: Zahn mit offenem Wurzelkanal und einer Unregelmäßigkeit am Grund des Wurzelkanals in Draufsicht.

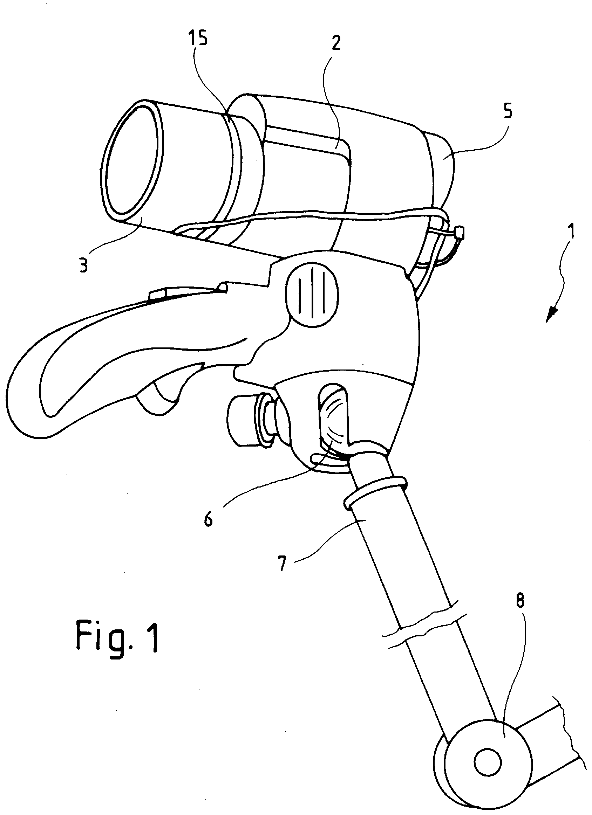

**Fig. 1** zeigt die erfindungsgemäße monokulare Vergrößerungsvorrichtung 1 zur visuellen Vergrößerung von Objekten in der Seitenansicht. Die in Form eines einrohrigen Fernglases ausgebildete optische Einheit 2 weist am proximalen Ende, das heißt an dem dem Betrachter zugewandten Ende, ein Okular 5 auf. Am distalen Ende beziehungsweise dem vom Betrachter abgewandten Ende der optischen Einheit 2 ist die optische Einheit 2 mit einer manuell bedienbaren Fokussiereinrichtung 15 versehen, die für eine Fokussierung bei einer variablen Veränderung des Abstandes zwischen dem zu untersuchenden Objekt und der optischen Einheit 2 im Bereich zwischen 20 cm bis 40 cm ausgebildet ist.
Die optische Einheit 2, insbesondere die Fokussiereinrichtung 15, ist nach einer alternativen Ausgestaltung mit elektrisch angetriebenen Stellmotoren versehen und mit einer Stromquelle elektrisch verbunden. Als Stromquelle dient dabei beispielsweise ein elektrischer Energiespeicher, wie eine Batterie, oder das öffentliche Stromnetz.
Die optische Einheit 2 ist dabei für eine bis zu 12fache Vergrößerung, insbesondere für eine 8fache bis 12fache Vergrößerung, für den Nahbereich vorgesehen.
Am distalen Ende ist die optische Einheit 2 in Richtung der optischen Achse vorteilhaft zudem mit einer Beleuchtungseinrichtung 3 versehen. Die Beleuchtungseinrichtung 3 ist, ebenso wie die optische Einheit 2 in diesem Bereich, zylinderförmig ausgebildet und starr, aber vorteilhaft lösbar mit der optischen Einheit 2 verbunden. Die Beleuchtungseinrichtung 3 ist dabei insbesondere hohlzylinderförmig ausgestaltet.
Die optische Einheit 2 ist wiederum mit Befestigungselementen 7 gekoppelt. Die Befestigungselemente 7 weisen bevorzugt Federarmhalterungen mit Scharniergelenken 8 für eine vertikale und horizontale Verschiebung der optischen Einheit 2 auf. Zudem sind die Befestigungselemente 7 vorteilhaft mit einem Kugelgelenk 6 ausgebildet, sodass die optische Einheit 2 zudem in alle Richtungen schwenkbar ist. Die Befestigungselemente 7 mit Kugelgelenk 6 und Scharniergelenken 8 der Federarmhalterungen ermöglichen damit eine richtungsunabhängige Verstellbarkeit der optischen Einheit 2. Die Objektivrichtung ist folglich in allen Richtungen, das heißt richtungsunbegrenzt, einstellbar. Ein weiterer Vorteil ist, dass die Befestigungselemente 7 zudem mit Feststelleinrichtungen versehen sind, um die gewünschte und eingestellte Position der optischen Einheit 2 in Bezug auf den Patienten und die behandelnde Person zu fixieren. Die Position der optischen Einheit 2 bleibt dadurch auch bei unerwarteten Erschütterungen erhalten. Die Befestigungselemente 7 stellen eine effektive und dabei einfache konstruktive Lösung für eine Halterung dar, mit welcher die optische Einheit 2 am Behandlungsstuhl mühelos montierbar ist.
Nach einer alternativen Ausgestaltung ist die optische Einheit 2 mit Beleuchtungseinrichtung 3 mittels der Befestigungselemente 7 auch direkt an der Operationsleuchte installierbar beziehungsweise in die Operationsleuchte integrierbar.

Die optische Einheit 2 ist in Verbindung mit den Befestigungselementen 7 sowie der Beleuchtungseinrichtung 3 zudem einfach zu bedienen, wobei die Möglichkeit der Einhandbedienung einen besonderen Vorteil darstellt. Die Bedienung erfordert nur eine sehr geringe Einarbeitungszeit für die bedienende Person.

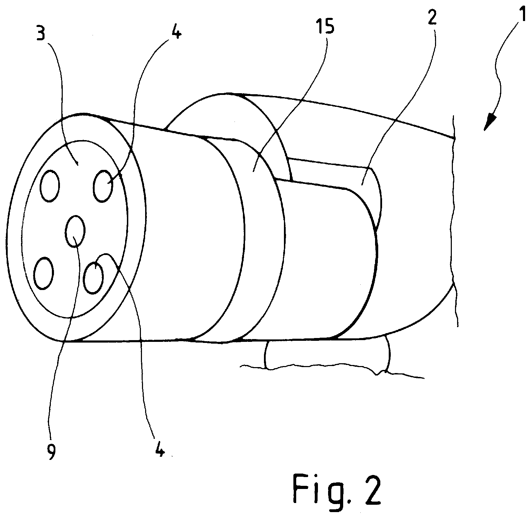

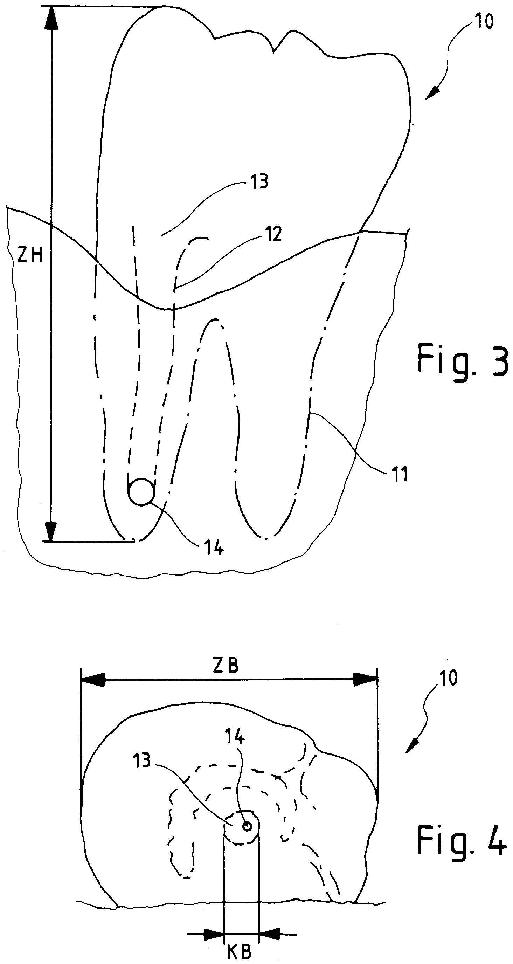

In **Fig. 2** sind die optische Einheit 2 und die Beleuchtungseinrichtung 3 mit optischem Durchlass 9 dargestellt. Der optische Durchlass 9 ist auf der optischen Achse der optischen Einheit 2 ausgerichtet.
Die hohlzylindrisch ausgebildete Beleuchtungseinrichtung 3 ist koaxial um den optischen Durchlass 9 positioniert und weist gleichmäßig am Umfang ringförmig verteilt angeordnete LED 4 auf. Die LED 4 sind dabei in ihrer Gesamtheit ebenfalls koaxial ausgerichtet, das heißt alle LED 4 sind im gleichen Abstand von der optischen Achse entfernt. Dabei sind bevorzugt mindestens vier LED 4 vorgesehen, wobei auch eine größere Anzahl an LED 4 möglich ist, die in bestimmten Kombinationen untereinander zuschaltbar sind. Die LED 4 erzeugen vorteilhaft ein zur optischen Achse der optischen Einheit 2 konzentrisches Beleuchtungsmuster mit sehr guter Ausleuchtung der zu untersuchenden Objekte, sodass die herkömmlichen Operationsleuchten ersetzt werden können.
Die erfindungsgemäße monokulare Vergrößerungsvorrichtung 1 mit koaxialer, konzentrischer Beleuchtung eignet sich dabei im zahnmedizinischen Bereich insbesondere zur Inspektion von Wurzelkanälen und Zahnzwischenräumen. Die tiefen und engen Zahnstrukturen sind nahezu schattenfrei ausleuchtbar. **Fig. 3** zeigt einen zu untersuchenden Zahn 10 mit angedeuteter äußerer Zahnkontur 11 und einem Wurzelkanal 12 in Seitenansicht. Der Zahn 10 weist beispielsweise eine Zahnhöhe ZH von 18 mm auf, wobei der Wurzelkanal 12 mit einem Durchmesser in der Größenordnung von 1 mm und einer Tiefe von etwa 12 mm bis 13 mm ausgebildet ist.
Im unteren Bereich des Wurzelkanals 12 ist eine zu untersuchende Unregelmäßigkeit 14 angedeutet. Unter Unregelmäßigkeiten 14 innerhalb des Wurzelkanals 12 von einem Zahn 10 sind beispielsweise der Kanalverlauf, insbesondere eine Kanalkrümmung oder versteckte Abzweigungen, Zementreste, Karies oder Ähnliches zu verstehen. Auch die Nerven des Zahnes 10 sind mit der erfindungsgemäßen Vergrößerungsvorrichtung 1 innerhalb des Wurzelkanals 12 leicht sichtbar.

**Fig. 4** zeigt in Analogie zu Fig. 3 den Zahn 10 mit offenem Wurzelkanal 12 sowie einer Unregelmäßigkeit 14 am Grund des Wurzelkanals 12 in der Draufsicht.
Der Zahn 10 weist beispielsweise eine Zahnbreite ZB von 10 mm auf. Der Kanaleingang 13 ist mit einer Kanalbreite KB von etwa 0,7 mm ausgebildet. Durch die Verwendung der erfindungsgemäßen Vergrößerungsvorrichtung 1 mit optischer Einheit 2 und Beleuchtungseinrichtung 3 zur visuellen Vergrößerung sowie Sichtbarmachung von Objekten für den Nahbereich lassen sich insbesondere tiefe Wurzelkanäle, aber auch Zahnzwischenräume einfach und effektiv untersuchen.

Generell ist es durch die erfindungsgemäße Realisierung der Vergrößerungsvorrichtung 1 zur visuellen Vergrößerung von Objekten zur Verwendung in der Medizin, insbesondere der Zahnmedizin und der Hals-Nasen-Ohren-Medizin, möglich, von der Verwendung sehr aufwendiger Operationsmikroskope und zusätzlicher Operationsleuchten abzusehen.

### LISTE DER BEZUGSZEICHEN

- 1: Vergrößerungsvorrichtung
- 2: optische Einheit
- 3: Beleuchtungseinrichtung
- 4: LED
- 5: Okular
- 6: Kugelgelenk
- 7: Befestigungselement
- 8: Scharniergelenk
- 9: optischer Durchlass
- 10: Zahn
- 11: äußere Zahnkontur
- 12: Wurzelkanal
- 13: Kanaleingang
- 14: Unregelmäßigkeit
- 15: Fokussiereinrichtung

- ZH: Zahnhöhe
- ZB: Zahnbreite
- KB: Kanalbreite

## Patentansprüche

1. Medizinische Vergrößerungsvorrichtung (1), aufweisend eine optische Einheit (2) mit einer optischen Achse, eine Beleuchtungseinrichtung (3) und Befestigungselemente (7), wobei
- die optische Einheit (2) am proximalen Ende ein Okular (5) aufweist, welches monokular ausgebildet ist,
- die Beleuchtungseinrichtung (3)
- hohlzylinderförmig ausgebildet ist,
- am distalen Ende mit der optischen Einheit (2) verbunden ist und
- koaxial zur optischen Achse, ein konzentrisches Beleuchtungsmuster erzeugend, angeordnet ist,
- die Befestigungselemente (7) mit der optischen Einheit (2) verbindbare derart ausgebildete Mittel umfassen, dass die optische Einheit (2) mit Okular (5) und Beleuchtungseinrichtung (3) richtungsunabhängig verstellbar - in horizontaler und vertikaler Richtung verschiebbar sowie in alle Richtungen schwenkbar - ist.

2. Medizinische Vergrößerungsvorrichtung (1) nach Anspruch 1, **dadurch gekennzeichnet, dass** die Beleuchtungseinrichtung (3) koaxial und ringförmig um die optische Achse angeordnete, gleichmäßig am Umfang verteilte LED's (4) aufweist.

3. Medizinische Vergrößerungsvorrichtung (1) nach Anspruch 1 oder 2, **dadurch gekennzeichnet, dass** elektrisch angetriebene Stellmotoren zur
Lageänderung vorgesehen und mit einer Stromquelle elektrisch verbunden ausgebildet sind.

4. Medizinische Vergrößerungsvorrichtung (1) nach einem der Ansprüche 1 bis 3, **dadurch gekennzeichnet, dass** die optische Einheit (2) und/oder die Beleuchtungseinrichtung (3) als Stromquelle einen elektrischen Energiespeicher aufweisen oder mit dem Stromnetz verbunden ausgebildet sind.

5. Medizinische Vergrößerungsvorrichtung (1) nach einem der Ansprüche 1 bis 4, **dadurch gekennzeichnet, dass** die optische Einheit (2) am proximalen Ende mit einer Bilderfassungsvorrichtung erweiterbar oder mit dem Okular (5) austauschbar ausgebildet ist.

6. Medizinische Vergrößerungsvorrichtung (1) nach einem der Ansprüche 1 bis 5, **dadurch gekennzeichnet, dass** die optische Einheit (2) eine Fokussiereinrichtung (15) aufweist, wobei die Fokussierung im Bereich zwischen 20 cm und 40 cm an der optischen Einheit (2) einstellbar ausgebildet ist.

7. Medizinische Vergrößerungsvorrichtung (1) nach einem der Ansprüche 1 bis 6, **dadurch gekennzeichnet, dass** die optische Einheit (2) mit einer bis zu 12fachen Vergrößerung für den Nahbereich ausgebildet ist.

8. Medizinische Vergrößerungsvorrichtung (1) nach einem der Ansprüche 1 bis 7, **dadurch gekennzeichnet, dass** die optische Einheit (2) mit einer 8fachen bis 12fachen Vergrößerung für den Nahbereich ausgebildet ist.

9. Medizinische Vergrößerungsvorrichtung (1) nach einem der Ansprüche 1 bis 8, **dadurch gekennzeichnet, dass** die Mittel der Befestigungselemente (7) eine Federarmhalterung mit Scharniergelenk (8), ein Kugelgelenk (6) und Feststelleinrichtungen umfassen.

10. Medizinische Vergrößerungsvorrichtung (1) nach einem der Ansprüche 1 bis 9, **dadurch gekennzeichnet, dass** die optische Einheit (2) mit der Beleuchtungseinrichtung (3) mittels der Befestigungselemente (7) an einem Behandlungsstuhl montierbar ausgebildet ist.

## Claims

1. Medical magnifying device (1), having an optical unit (2) with an optical axis, illumination device (3) and fixing elements (7), whereby
- the optical unit (2) has an eyepiece (5) at the proximal end which has a monocular design,
- the illumination device (3)
- is designed with a hollow cylindrical shape,
- is connected at the distal end to the optical unit (2) and
- is arranged coaxial to the optical axis generating a concentric illumination pattern,
- the fixing elements (7) comprise means connectable to the optical unit (2) and designed in such a way that the optical unit (2) with eyepiece (5) and illumination device (3) is adjustable regardless of direction - moveable in a horizontal and vertical direction and capable of being swivelled in all directions.

2. Medical magnifying device (1) in accordance with Claim 1, **characterised in that** the illumination device (3) has LEDs (4) arranged coaxially and in a ring shape around the optical axis, distributed evenly around the circumference.

3. Medical magnifying device (1) in accordance with Claim 1 or 2, **characterised in that** electrically driven actuators for changing position are provided and designed electrically connected to a power source.

4. Medical magnifying device (1) in accordance with one of Claims 1 to 3, **characterised in that** the optical unit (2) and/or the illumination device (3) have an electric energy store as a power source or are designed connected to the mains.

5. Medical magnifying device (1) in accordance with one of Claims 1 to 4, **characterised in that** the optical unit (2) is designed capable of being extended at the proximal end with an image capture device or designed with the eyepiece (5) capable of being exchanged.

6. Medical magnifying device (1) in accordance with one of Claims 1 to 5, **characterised in that** the optical unit (2) has a focusing device (15), whereby the focusing is designed to be adjustable in the range 20 cm to 40 cm on the optical unit (2).

7. Medical magnifying device (1) in accordance with one of Claims 1 to 6, **characterised in that** the optical unit (2) is designed with a magnification of up to 12x for close-up views.

8. Medical magnifying device (1) in accordance with one of Claims 1 to 7, **characterised in that** the optical unit (2) is designed with an 8x to 12x magnification for close-up views.

9. Medical magnifying device (1) in accordance with one of Claims 1 to 8, **characterised in that** the means of the fixing elements (7) include a spring arm holder with a hinge joint (8), a ball joint (6) and locking devices.

10. Medical magnifying device (1) in accordance with one of Claims 1 to 9, **characterised in that** the optical unit (2) with the illumination device (3) is designed capable of being mounted on a treatment chair by means of the fixing elements (7).

## Revendications

1. Dispositif médical de grossissement (1), comprenant une unité optique (2) avec un axe optique, un dispositif d'éclairage (3) et des éléments de fixation (7), **caractérisé en ce que**
- l'unité optique (2) est pourvue, à l'extrémité proximale, d'un oculaire (5) monoculaire,
- le dispositif d'éclairage (3)
présente une forme de cylindre creux,
- l'extrémité distale présente un raccordement à l'unité optique (2) et
- un modèle d'éclairage concentrique est généré sur le plan coaxial par rapport à l'axe optique,
- les éléments de fixation (7) de l'unité optique (2) constituent un moyen de liaison tel que l'unité optique (2) avec oculaire (5) et dispositif d'éclairage (3) est réglable indépendamment de la direction - coulissable à l'horizontale et à la verticale et inclinable dans toutes les directions.

2. Dispositif médical de grossissement (1) selon la revendication 1, **caractérisé en ce que** le dispositif d'éclairage (3) se présente sur le plan coaxial et sous la forme annulaire de LED (4) disposées sur l'axe optique (4) à intervalle réguliers.

3. Dispositif médical de grossissement (1) selon la revendication 1 ou 2, **caractérisé en ce que** des servomoteurs électriques sont prévus pour modifier la position et raccordés à une alimentation électrique.

4. Dispositif médical de grossissement (1) selon les revendications 1 à 3, **caractérisé en ce que** l'unité optique (2) et/ou le dispositif d'éclairage (3) sont dotés d'une batterie électrique ou raccordés au secteur.

5. Dispositif médical de grossissement (1) selon les revendications 1 à 4, **caractérisé en ce que** l'unité optique (2) peut être étendue, à l'extrémité proximale, par un dispositif d'acquisition d'images, ou bien remplacée par l'oculaire (5).

6. Dispositif médical de grossissement (1) selon les revendications 1 à 5, **caractérisé en ce que** l'unité optique (2) est dotée d'un dispositif de focalisation (15), la focalisation étant réglable sur une plage comprise entre 20 cm et 40 cm sur l'unité optique (2).

7. Dispositif médical de grossissement (1) selon les revendications 1 à 6, **caractérisé en ce que** l'unité optique (2) peut effectuer un grossissement jusqu'à 12 fois pour les courtes portées.

8. Dispositif médical de grossissement (1) selon les revendications 1 à 7, **caractérisé en ce que** l'unité optique (2) peut effectuer un grossissement de 8 fois à 12 fois pour les courtes portées.

9. Dispositif médical de grossissement (1) selon les revendications 1 à 8, **caractérisé en ce que** le moyen composé des éléments de fixation (7) englobe un support pour bras à ressort avec charnière (8), rotule (6) et dispositifs de blocage.

10. Dispositif médical de grossissement (1) selon les revendications 1 à 9, **caractérisé en ce que** l'unité optique (2) avec dispositif d'éclairage (3) peut être montée à l'aide des éléments de fixation (7),par ex. sur un fauteuil de dentiste.
